# EUROPÄISCHE PATENTANMELDUNG

(11) **EP 1 714 632 A1**
(43) Veröffentlichungstag der Anmeldung: **25.10.2006**
(21) Anmeldenummer: 06007347.5
(22) Anmeldetag: 07.04.2006
(51) Int. Cl.: A61K 6/02, A61K 6/087

(54) **Verwendung von Butylenterephtalat zur Herstellung thermoplastischen Zahnersatzes**

(30) Priorität: 20.04.2005 DE 102005018450
(71) Anmelder: Heraeus Kulzer GmbH, 63450 Hanau (DE)
(72) Erfinder: Renz, Karl-Heinz, 71131 Jettingen (DE)
(74) Vertreter: Kühn, Hans-Christian

(57) **Zusammenfassung**

Es werden die Verwendung zyklischen, oligomeren Butylenterephthalats (CBT™) zur Herstellung thermoplastischen Zahnersatzes oder zur Herstellung von Ausgangsmaterialien für thermoplastischen Zahnersatz beschrieben, ferner CBT™enthaltende Ausgangsmaterialien zur Herstellung thermoplastischen Zahnersatzes, sowie Verfahren zur Herstellung thermoplastischen Zahnersatzes.

## Beschreibung

Die vorliegende Erfindung betrifft die Verwendung von Butylenterephthalat zur Herstellung thermoplastischen Zahnersatzes, insbesondere Prothesen, künstlicher Zähne, Kronen oder Brücken, zur Herstellung von Ausgangsmaterialien für thermoplastischen Zahnersatz, die Ausgangsmaterialien, sowie Verfahren zur Herstellung thermoplastischen Zahnersatzes unter Verwendung von Butylen-terephthalat.

Man unterscheidet drei Hauptmaterialklassen zur Herstellung von totalprothetischen Arbeiten: auf Polymethylmethacrylat (PMMA) basierende Zweikomponentenmaterialien; PMMA-freie, heißhärtende Materialien sowie thermoplastisch verarbeitbare Spritzgießmassen.

Thermoplastischer Zahnersatz kann im Spritzgussverfahren aus Polyacetalen oder Mischungen aus PMMA/Polyamid hergestellt werden. Nachteile dabei sind die sehr hohe Verarbeitungstemperatur und schwierige Prozessbedingungen, wie hohe Materialviskosität und dadurch bedingte sehr hohe Verarbeitungsdrücke. Zahnzwischenräume lassen sich z.B. nicht einfach ausfüllen.

Cyclische Oligomere von Butylenterephthalat sind seit langem bekannt [z.B. Mueller, F. J. et al. "Synthesis of Cyclic Oligomers of Butylene Terephthalate" Makromol. Chem.184, 2487-95 (1983)].

Die Firma Cyclics Corporation, Schenectady, New York, USA, bietet cyclisches, oligomeres Butylenterephtalat (CBT™) zum Verkauf an. Die Herstellung erfolgt über handelsübliches Polybutylenterephtalat (PBT). CBT™ -Harze schmelzen relativ niedrig, sind sehr fließfähig und ergeben ausgehärtet Kunststoffmaterial mit guten mechanischen Eigenschaften, das zu komplexen Teilen geformt werden kann. Darüber hinaus kann das Material im Gegensatz zu traditionellen Epoxyharzen leicht recycled werden. Material und Herstellung sind z.B. in US 6,420,048 B1, US 6,420,047 B1 und US 6,369,157 B1 beschrieben.

Wird CBT™ mit Initiatoren abgemischt, erfolgt eine Rückreaktion zu PBT. CBT™ hat den Vorteil, dass es eine sehr viel geringere Viskosität als PBT besitzt, und so feine Kanäle besser durchdringen kann. Dies kann bei der Herstellung von Zahnersatz mit überraschend guten Resultaten genutzt werden. So können beispielsweise Zahnzwischenräume durch ein nahezu zu 100% thermoplastisches Material ausgefüllt werden. Die bisher auftretenden Nachteile thermoplastischer Zahnersatzmaterialien werden dadurch ausgeglichen.

Die vorliegende Erfindung betrifft somit die
Verwendung zyklischen, oligomeren Butylenterephthalats zur Herstellung thermoplastischen Zahnersatzes oder zur Herstellung von Ausgangsmaterialien für thermoplastischen Zahnersatz,
insbesondere zur Herstellung von Prothesen (auch faserverstärkt), künstlicher Zähne, Kronen oder Brücken (auch faserverstärkt), sowie von Ausgangsmaterialien dafür.

Der Gegenstand der Erfindung stellt eine günstige Alternative zur Verarbeitung von thermoplastischen Zahnersatzmaterialien im "dünnflüssigen" Zustand - also im wesentlichen bei hohen Temperaturen - dar. Die oben erwähnten Nachteile des Arbeitens mit thermoplastischem Material werden weitestgehend vermieden.

Die Erfindung betrifft auch Ausgangsmaterial zur Herstellung thermoplastischen Zahnersatzes, enthaltend

| | |
|---|---|
| CBT™ | 50- 99,9 Gew.-%, |
| Polycaprolacton | 0 - 20 Gew.-%, |
| Initiatorkomponenten | 0,001 - 2 Gew.-%, |
| Anorganische Füllstoffe | 0 - 50 Gew.-%, |
| Organische Füllstoffe | 0 - 50 Gew.-%. |

Zusätzlich können weitere übliche Zusatzstoffe, wie etwa Farbstoffe oder Pigmente enthalten sein. Als Initiatoren kommen vornehmlich Organozinn- oder Titanatverbindungen in Frage.

Materialtechnische Vorteile im Vergleich zu herkömmlichen in der Zahntechnik verwendeten thermoplastischen Materialien sind eine sehr ausgewogene Einstellbarkeit von Steifigkeit und Schlagzähigkeit. So können relativ steife Formkörper mit hoher Schlagzähigkeit erzeugt werden.

Die Erfindung betrifft auch Verfahren zur Herstellung thermoplastischen Zahnersatzes, insbesondere Zahnprothesen, mit den Schritten
- Modellierung des/der zu ersetzenden Teils/Teile in Wachs;
- Einbetten des Wachsmodells;
- Härten des Einbettmaterials;
- Entfernen des Wachses;
- Einbringen einer flüssigen CBT™-Mischung in den entstandenen Hohlraum;
- Härten der CBT™ Mischung.

Entsprechend werden z.B. Zähne nach herkömmlicher Vorgehensweise in Wachs aufgestellt, dann nach Umfassen der Zähne mit einem Einbettmaterial das Wachs entfernt und der entstehende Hohlraum durch eine flüssige CBT™-Mischung ersetzt und diese ausgehärtet.

### Beispiel

Eine Zusammensetzung enthaltend

| | |
|---|---|
| CBT™ | 99,7 Gew.-% |
| entsprechende Initiatorkomponenten | 0,3 % |

wird bei ca. 170 °C in Platten gegossen und bei ca. 200 °C ausgehärtet.

Es ergibt sich PBT mit folgenden Eigenschaften

| | |
|---|---|
| Biegefestigkeit (ISO 1567): | 85 MPa. |
| Biegemodul (ISO 1567): | 2500 MPa. |
| Schlagzähigkeit (ISO 1567, Amendment 1): | 1,5 MPa. |

## Patentansprüche

1. Verwendung zyklischen, oligomeren Butylenterephthalats zur Herstellung thermoplastischen Zahnersatzes oder zur Herstellung von Ausgangsmaterialien für thermoplastischen Zahnersatz.

2. Verwendung nach Anspruch 1 zur Herstellung von Prothesen, künstlichen Zähnen, Kronen oder Brücken.

3. Ausgangsmaterial zur Herstellung thermoplastischen Zahnersatzes, enthaltend
| | |
|---|---|
| zyklisches, oligomeres Butylenterephthalat | 50 - 99,9 Gew.-%, |
| Polycaprolacton | 0 - 20 Gew.-%, |
| Initiatorkomponenten | 0,001 - 2 Gew.-%, |
| Anorganische Füllstoffe | 0 - 50 Gew.-%, |
| Organische Füllstoffe | 0 - 50 Gew.-%. |

4. Verfahren zur Herstellung thermoplastischen Zahnersatzes, mit den Schritten:
• Modellierung des/der zu ersetzenden Teils/Teile in Wachs;
• Einbetten des Wachsmodells;
• Härten des Einbettmaterials;
• Entfernen des Wachses;
• Einbringen einer flüssigen zyklischen, oligomeren Butylenterephthalat-Mischung gemäß Anspruch 3 in den entstandenen Hohlraum;
• Härten der zyklischen, oligomeren Butylenterephthalat-Mischung.

5. Verfahren nach Anspruch 4 zur Herstellung von Zahnprothesen.
